# EUROPÄISCHE PATENTSCHRIFT

(11) **EP 1 807 032 B1**
(45) Veröffentlichungstag und Bekanntmachung des Hinweises auf die Patenterteilung: **05.07.2017**
(21) Anmeldenummer: 05810769.9
(22) Anmeldetag: 02.11.2005
(51) Int. Cl.: A61F 13/02, A61F 13/00, A61M 1/00

(54) **MEHRKOMPONENTENVERBAND ZUR WUNDBEHANDLUNG DES MENSCHLICHEN ODER TIERISCHEN KÖRPERS UNTER EINSATZ VON UNTERDRUCK**
MULTI-COMPONENT DRESSING FOR WOUND TREATMENT ON THE HUMAN OR ANIMAL BODY WITH APPLICATION OF REDUCED PRESSURE
BANDAGE A PLUSIEURS COMPOSANTS SERVANT A TRAITER UNE PLAIE AFFECTANT LE CORPS D'UN ETRE HUMAIN OU D'UN ANIMAL AU MOYEN D'UNE PRESSION NEGATIVE

(30) Priorität: 02.11.2004 DE 202004017052 U
(43) Veröffentlichungstag der Anmeldung: 18.07.2007
(60) Teilanmeldung: 14173339.4
(73) Patentinhaber: BSN medical GmbH, 20253 Hamburg (DE)
(72) Erfinder: RIESINGER, Birgit, 48149 Münster (DE)
(74) Vertreter: Jostarndt Patentanwalts-AG
(86) Internationale Anmeldenummer: PCT/EP2005/011701
(87) Internationale Veröffentlichungsnummer: WO 2006/048246

(56) Entgegenhaltungen:
- EP-A- 1 177 781
- WO-A-01/10363
- DE-A1- 10 059 439
- US-A- 4 382 441
- US-A- 5 549 584
- US-A- 5 636 643
- US-A1- 2002 065 494
- US-B1- 6 174 306

## Beschreibung

Die Erfindung betrifft einen Mehrkomponentenverband zur Wundbehandlung des menschlichen oder tierischen Körpers unter Einsatz von Unterdruck, aufweisend:
- ein Wundabdeckungselement zur Anbringung an Hautoberfläche,
- wenigstens eine Anschlussstelle, die mit dem Wundraum in Kontakt steht und über welche die im Wundraum befindlichen Stoffe evakuiert werden können.

Ein derartiger Mehrkomponentenverband ist der US 5636643 bekannt. Nachteilig bei dem bekannten Mehrkomponentenverband ist, dass das Wundsekret ausschliesslich über eine Schlauchleitung dem Wundgebiet zu entziehen ist.

Aufgabe der Erfindung ist, einen neuartigen Mehrkomponentenverband zu konzipieren, bei dem die Wundsekrete im Wundgebiet verbleiben können, ohne dass diese Wundsekrete ihren schädigenden Eigenschaften im Wundgebiet nachkommen.

Diese Aufgabe ist durch einen Mehrkomponentenverband gemäss Anspruch 1 gelöst. Als Polymere können alle bekannten Polymere, jedoch vorzugsweise solche aus der Gruppe der Natriumpolyacrylate gewählt sein.

Der Mehrkomponentenverband ist mit wenigstens einen umhüllten Absorptionskörper versehen welcher wenigstens eine Lage eines mit superabsorbierenden Partikeln durchsetzten Textilabschnittes aufweist.

Der Absorptionskörper kann von einer flüssigkeitsdurchlässigen Hülle umgeben sein, welche wiederum Poren aufweist, deren Grösse die der superabsorbierenden Partikeln im Wesentlichen nicht überschreitet.

Der außen generierte Unterdruck wird über eine Schlauchleitung oder gegebenenfalls über einen Saugkopf in den Wundraum geführt, wo er gewünschte Synergien mit den Polymeren unterstützt.

Die Erfindung ist anhand der Zeichnung näher erläutert. Die Figuren zeigen:
- Figuren 1a bis 1d: einen an die Haut des Patienten um eine Wunde herum angeklebten Mehrkomponentenverband, in einem schematischen Schnitt; angeklebten
- Fig. 2: eine andere Ausführungsform des Mehrkomponentenverbandes, in einem schematischen Schnitt;
- Fig. 3: den Mehrkomponentenverband gemäß der Fig. 1, jedoch mit zwei Anschlußstellen, ebenfalls in einem schematischen Schnitt.

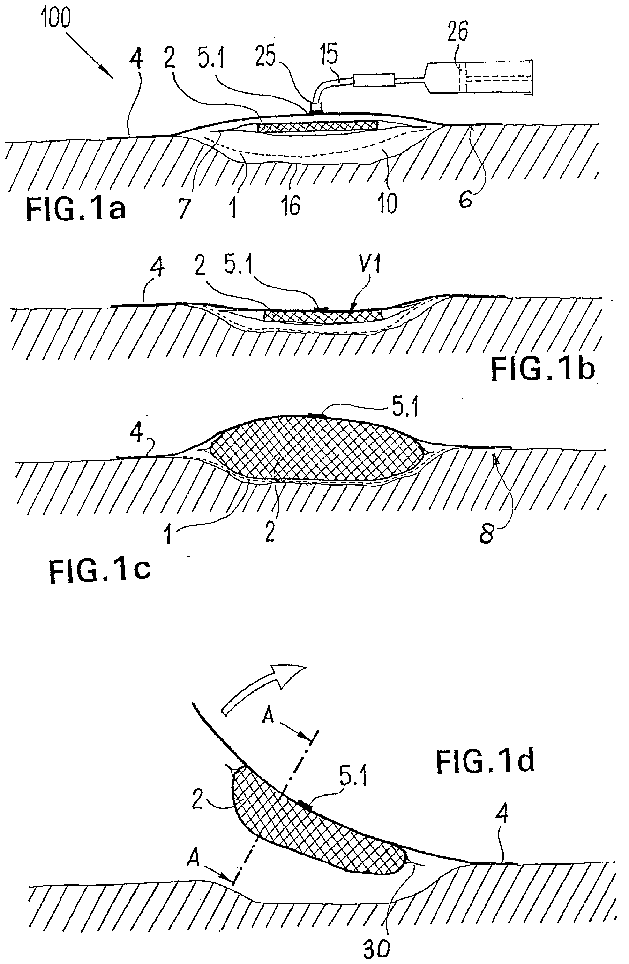

Die Figuren 1a bis 1d zeigen einen Mehrkomponentenverband 100 zur Wundbehandlung unter Einsatz von Unterdruck, bestehend aus einem folienartigen Wundabdeckungselement 4, einem optionalen schleimhautfreundlichen Folienelement 1 und einem dazwischen liegenden Absorptionskörper 2. Der Mehrkomponentenverband ist in Draufsicht auf ihre Flachseite etwa rechteckig und weist abgerundete Ecken (nicht dargestellt) auf.

Das aus einer flüssigkeitsundurchlässigen, transparenten Folie bestehende Wundabdeckungselement 4 ist relativ steif, d. h. es schrumpft nicht im nicht benutzten und im am Körper des Patienten angelegten Zustand. Das Wundabdeckungselement 4 ist an seiner Peripherie 8 mit einer Klebefläche 6 zum Ankleben des Mehrkomponentenverbandes an die Haut des Patienten versehen.

Der Absorptionskörper 2 besteht aus einer Lage 22 eines vliesartigen, textilen Materials, das Cellulosefasern umfaßt und mit Superabsorberteilchen (Super-Absorbing-Polymers, SAP), im vorliegenden Fall mit Natriumacrylat-Acrylsäure-Polymerisat durchsetzt ist. Außerdem ist der Absorptionskörper 2 mit nanokristallinen, silberhaltigen Substanzen angereichert, die mikrobizid wirken. Die Cellulosefasern wirken als Zwischenspeicher der spontan beaufschlagten Flüssigkeitsmengen und als eine Art Transportmittel, mit dem die Wundsekrete bis zum Superabsorber hin gelangen.

Die Lage 22 ist von einer flüssigkeitsdurchlässigen, ebenfalls textilen, mit einer umlaufenden Ultraschallnaht 7 verschweißten Hülle 11 umgeben. Wie insbesondere der Fig. 1d zu entnehmen ist, weist die Hülle 11 einen peripheren Überstand 30 an Hüllenmaterial auf, der zwischen der Ultraschallnaht 7 und einem äußersten Umfang der Hülle 11 liegt. Die Aufgabe des Überstandes 30 ist, die Wunde vor der schmerzhaften Berührung mit der Naht zu verhindern.

Das der Wunde zugewandte Folienelement 1 ist aus einem flüssigkeitsdurchlässigen, hauchdünnen, schleimhautfreundlichen Material angefertigt. Das Folienelement 1 trägt auch dem Schutz vor Berührung mit der Ultraschallnaht 7 bei.

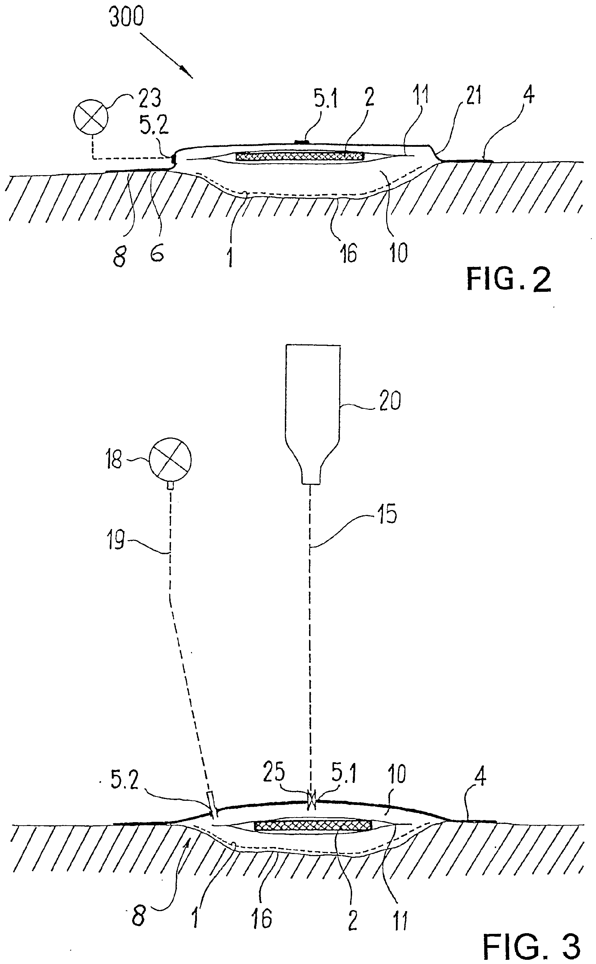

Ferner ist am Wundabdeckungselement 4 eine Anschlußstelle 5.1 zur Gasevakuierung und zur Kontrolle des Unterdrucks eingebracht. Gemäß den Figuren 1a bis 1d ist die Anschlußstelle 5.1 etwa mittig angeordnet. Sie kann jedoch an beliebiger Stelle am Wundabdeckungselement liegen, beispielsweise in der Nähe der Peripherie 8, wie in der Fig. 3 gezeigt worden ist.

Der in den Wundraum 10 einzulegende Absorptionskörper 2 weisen ein Anfangsvolumen V1 auf, das sich im Laufe des Absorptionsprozesses vergrößert und ein Endvolumen V2 annimmt, mit dem sich der Wundraum 10 und damit die jeweiligen Wunddefekte während des Quellvorgangs auffüllen lassen.

In das Material des Absorptionskörpers 2 sind den Wundheilungsprozess beeinflussende 1 Wirksubstanzen, beispielsweise nanokristalline Silberpartikeln appliziert.

Wie die Fig. 1d zeigt, ist der Absorptionskörper 2 ganzflächig mit einem Wundabdeckungselement 4 unter Belassung einer freien Peripherie 8 am Wundabdeckungselement verklebt.

Der Mehrkomponentenverband gemäß der Fig. 3 weist zwei Anschlußstellen 5.1, 5.2 auf, von denen die eine, mittige zur Luftevakuierüng und die zweite, seitliche zur Unterdruckkontrolle dient. An die mittige Anschlußstelle 5.1 ist über eine Schlauchleitung 15 eine Vakuumflasche 20, dagegen an die seitliche Anschlußstelle 5.2 ebenfalls über einen Verbindungsschlauch 19 eine Intervall-Schaltung 18 angeschlossen ist. Die Beschreibung der Fig. 7 bezieht sich selbstverständlich auf die an die Haut des menschlichen Patienten oder des Tieres angeklebte Vorrichtung.

### Funktion:

Eine chronische Wunde 16 wird durch das Ankleben des Mehrkomponentenverbandes 100 gemäß der Fig. 1 auf die Haut des Patienten vollständig bedeckt. Vorher wurde ein nicht gezeigtes Abziehfolienelement entfernt, das eine periphere Klebefläche 6 an der Unterseite des Wundabdeckungselementes 4 freigibt. Mit einer sterilisierten Pinzette (nicht dargestellt) wurde zuerst das schleimhautfreundliche Folienelement 1, dann der flache Absorptionskörper 2 samt Hülle 11 vorsichtig auf die Wundoberfläche gelegt und erst danach das Wundabdeckungselement 4 um die Wunde herum angeklebt. Durch das Ankleben der Vorrichtung auf die Haut ist ein Wundraum 10 zwischen dem Wundabdeckungselementes 4 und der Wundoberfläche entstanden. An die mittige, mit einem einfachen Ventil 25 (vgl. Fig. 1a) versehene Anschlußstelle 5.1 wurde über die vorgenannte Schlauchleitung 15 eine ärztliche Injektionsspritze 26 angeschlossen. Da der Raum 10 abgedichtet ist, können mit Hilfe der Injektionsspritze die im Raum befindlichen Gase evakuiert werden. Den Zustand zeigt die Fig. 1b. Die flachen Elemente der Vorrichtung liegen auf der Wundoberfläche an. Der inzwischen mit Hilfe eines nicht dargestellten Vakuum-Indikators bemessene Unterdruck betrug etwa 100 ml Hg. Hierbei kann die zylindrische Mantelfläche der Injektionsspritze mit einer entsprechenden, experimental definierten Unterdruck-Skala versehen sein.

Die aus der Wunde heraustretenden Wundsekrete gelangen in den Absorptionskörper 2 und bewirken eine langsam wachsende Kompression unterhalb des Wundabdeckungselementes 4. Nach dem Ansaugen von Wundsekreten nimmt das Volumen des Absorptionskörpers 2 stark zu (vgl. Fig. 1c).
Die verbrauchte Vorrichtung 100 wird jetzt durch das Anheben mit Hilfe der Pinzette vorsichtig aus dem Bereich der Wunde entfernt. Nach Bedarf kann auf die Wunde ein neuer Mehrkomponentenverband aufgeklebt werden.

## Patentansprüche

1. Mehrkomponentenverband (100) zur Wundbehandlung des menschlichen oder tierischen Körpers unter Einsatz von Unterdruck, aufweisend:
ein Wundabdeckungselement (4) zur Anbringung an der Hautoberfläche,
wenigstens eine Anschlussstelle (5), die mit dem Wundraum (10) in Kontakt steht,
und über welche die im Wundraum (10) befindlichen Stoffe evakuiert werden können,
**dadurch gekennzeichnet, dass**
dieser wenigstens einen umhüllten Absorptionskörper (2) mit wenigstens einer Lage (12) eines mit superabsorbierenden Partikeln durchsetzten Textilabschnittes (2) aufweist,
wobei die superabsorbierenden Partikel Polymere aufweisen,
wobei die absorbierten Wundsekrete an die Polymere gebunden im Wundraum bis zur Entfernung aus dem Wundraum verbleiben.

2. Mehrkomponentenverband nach Anspruch 1, **dadurch gekennzeichnet, dass** als Stoffe die im Wundraum befindlichen Gase evakuiert werden können.

3. Mehrkomponentenverband nach Anspruch 1 oder 2, **dadurch gekennzeichnet, dass** die Polymere vorzugsweise aus der Gruppe der Natriumpolyacrylate gewählt sind.

4. Mehrkomponentenverband nach einem der Ansprüche 1 bis 3, **dadurch gekennzeichnet, dass** der Absorptionskörper (2) in eine flüssigkeitsdurchlässige Hülle (11) eingefasst ist, welche Poren aufweist, deren Größe die der superabsorbierenden Partikeln im Wesentlichen nicht überschreitet.

5. Mehrkomponentenverband nach Anspruch 4, **dadurch gekennzeichnet, dass** der in den Wundraum (10) einzulegende Absorptionskörper (2) ein Anfangsvolumen (V1) aufweist, das sich im Laufe des Absorptionsprozesses vergrössert und ein Endvolumen (V2) annimmt, mit dem sich der Wundraum (10) und damit die jeweiligen Wunddefekte während des Quellvorgangs auffüllen lassen.

6. Mehrkomponentenverband nach einem der Ansprüche 1 bis 5, **dadurch gekennzeichnet, dass** die Lage (12) in Draufsicht auf ihre Flachseite eine Flächenausdehnung hat, die um 3% bis 90% kleiner ist als die der flachgelegten Hülle (11).

7. Mehrkomponentenverband nach einem der Ansprüche 1 bis 6, **dadurch gekennzeichnet, dass** dem Material des Absorptionskörpers (2) den Wundheilungsprozess beeinflussende (1) Wirksubstanzen, beispielsweise nanokristalline Silberpartikeln appliziert sind.

8. Mehrkomponentenverband nach einem der Ansprüche 1 bis 7, **dadurch gekennzeichnet, dass** der Absorptionskörper (2) ganzflächig mit einem Wundabdeckungselement (4) unter Belassung einer freien Peripherie (8) am Wundabdeckungselement verklebt ist.

## Claims

1. A multi-component dressing (100) for treating wounds on the human or animal body, using negative pressure, comprising:
a wound-covering element (4) to be applied to the surface of the skin, at least one connecting site (5) which is in contact with the wound space (10) and via which the substances present in the wound space (10) can be evacuated,
**characterized in that**
said multi-component dressing (100) has at least one sheathed absorption body (2) which has at least one layer (12) of a textile section (2) interspersed with superabsorbent particles,
whereby the superabsorbent particles contain polymers,
whereby the absorbed wound secretions are bound to the polymers in the wound space and remain there until their removal from the wound space.

2. The multi-component dressing according to claim 1, **characterized in that** the gases, as the substances present in the wound space, can be evacuated.

3. The multi-component dressing according to claim 1 or 2, **characterized in that** the polymers are preferably selected from the group of sodium polyacrylates.

4. The multi-component dressing according to one of claims 1 to 3, **characterized in that** the absorption body (2) is enclosed in a fluid-permeable sheath (11) which has pores whose size essentially does not exceed that of the superabsorbent particles.

5. The multi-component dressing according to claim 4, **characterized in that** the absorption body (2), which is to be placed into the wound space (10) has an initial volume (V1) that becomes enlarged over the course of the absorption process and that acquires a final volume (V2) with which the wound space (10) and thus the appertaining wound defects can be filled during the swelling process.

6. The multi-component dressing according to one of claims 1 to 5, **characterized in that**, as seen in a plan view of its flat side, the layer (12) has a surface area extension that is 3% to 90% smaller than that of the sheath (11) when the latter has been laid out flat.

7. The multi-component dressing according to one of claims 1 to 6, **characterized in that** active substances that have an effect on the wound healing process such as, for example, nano-crystalline silver particles are applied to the material of the absorption body (2).

8. The multi-component dressing according to one of claims 1 to 7, **characterized in that** the absorption body (2) is glued over its whole surface to a wound-covering element (4), leaving a free periphery (8) on the wound-covering element.

## Revendications

1. Bandage à plusieurs composants (100) servant à traiter une plaie affectant le corps d'un être humain ou d'un animal au moyen d'une pression négative, comportant :
un élément de recouvrement de plaie (4) destiné à être appliqué sur la surface cutanée,
au moins un point de jonction (5) qui est en contact avec l'espace de plaie (10) et vi lequel peuvent être évacuées les substances qui se trouvent dans l'espace de plaie (10),
**caractérisé en ce que**
celui-ci comporte au moins un corps absorbant enveloppé (2) avec au moins une couche (12) d'une portion de textile (2) parsemée de particules superabsortantes, les particules superabsorbantes comportant des polymères,
les sécrétions de plaie absorbées restant liées aux polymères dans l'espace de plaie jusqu'au retrait hors de l'espace de plaie.

2. Bandage à plusieurs composants selon la revendication 1, **caractérisé en ce que** peuvent être évacués, en tant que substances, les gaz qui se trouvent dans l'espace de plaie.

3. Bandage à plusieurs composants selon la revendication 1 ou 2, **caractérisé en ce que** les polymères sont choisis préférentiellement dans le groupe des polyacrylates de sodium.

4. Bandage à plusieurs composants selon l'une des revendications 1 à 3, **caractérisé en ce que** le corps absorbant (2) est contenu dans une enveloppe (11) perméable aux liquides qui présente des pores dont la taille ne dépasse sensiblement pas celle des particules superabsorbantes.

5. Bandage à plusieurs composants selon la revendication 4, **caractérisé en ce que** le corps absorbant (2) à poser dans l'espace de plaie (10) présente un volume initial (V1) qui augmente au cours du processus d'absorption et atteint un volume final (V2) permettant de remplir l'espace de plaie (10) et, par conséquent, les défectuosités dues à la plaie pendant l'opération de gonflage.

6. Bandage à plusieurs composants selon l'une des revendications 1 à 5, **caractérisé en ce que** la couche (12) présente, en vue de dessus sur son côté plat, une superficie de 3% à 90% inférieure à celle de l'enveloppe mise à plat (11).

7. Bandage à plusieurs composants selon l'une des revendications 1 bis 6, **caractérisé en ce que** sont appliquées à la matière du corps absorbant (2) des substances actives influençant (1) le processus de guérison de la plaie, par exemple, des particules d'argent nanocristallines.

8. Bandage à plusieurs composants selon l'une des revendications 1 à 7, **caractérisé en ce que** le corps absorbant (2) est collé sur toute sa surface à un élément de recouvrement de plaie (4) tout en laissant une périphérie libre (8) sur l'élément de recouvrement de plaie.
